# EUROPÄISCHE PATENTANMELDUNG

(11) **EP 3 050 539 A1**
(43) Veröffentlichungstag der Anmeldung: **03.08.2016**
(21) Anmeldenummer: 15152597.9
(22) Anmeldetag: 27.01.2015
(51) Int. Cl.: A61F 2/34, A61F 2/30, A61F 2/32

(54) **Revisionsendoprothese**

(71) Anmelder: AQ Implants GmbH, 22926 Ahrensburg (DE)
(72) Erfinder: Drenckhan, Sylke, 23556 Lübeck (DE); Hartung, Ingo, 23556 Lübeck (DE)
(74) Vertreter: Grünecker Patent- und Rechtsanwälte PartG mbB

(57) **Zusammenfassung**

Die vorliegende Erfindung betrifft eine Revisionsendoprothese mit einer Pfanne (10). Zur besseren Versorgung von Patienten, deren Endoprothesen-Pfanne zumindest einmal, gegebenenfalls auch mehrmalig ausgetauscht worden ist und die ausgedehnte Knochendefekte und Bandinstabilitäten haben, wird mit der vorliegenden Erfindung eine Revisionsendoprothese mit einer Pfanne vorgeschlagen, die eine innere Aufnahme zur verschwenkbaren Lagerung des Oberschenkelknochens ausformt und eine sphärische Außenfläche (32) hat, die zumindest in einer Ebene nicht rotationssymmetrisch ist, wobei zusätzlich ein Einsatz mit einer sphärischen Außenfläche zur kugeligen Lagerung innerhalb der inneren Aufnahme und einer sphärischen Innenfläche zur kugeligen Lagerung eines Kugelkopfes (40), der mit dem Oberschenkelknochen verbunden ist, vorgesehen ist, sodass die relative Beweglichkeit zwischen Oberschenkelknochen durch zwei Artikulationsflächen erreicht wird.

## Beschreibung

Die vorliegende Erfindung betrifft eine Revisionsendoprothese. Solche Endoprothesen sind beispielsweise aus der DE 10 2006 002 210, der DE 197 01 778 bzw. der DE 201 05 862 bekannt. Revisionsendoprothesen zeichnen sich dadurch aus, dass die Pfanne, d.h. derjenige Körper der Endoprothese, der hüftseitig also acetabular mit dem Knochen befestigt wird, eine nicht rotationssymmetrische, d.h. ovale Grundfläche hat. Damit wird dem Umstand Rechnung getragen, dass beim Einsatz der Revisionspfanne, die als Ersatz für eine zuvor bereits implantierte Pfanne für die erstmalige Hüftprothetik des entsprechend geschädigten Hüftgelenkes implantiert wird, das beim Versagen der ersten ursprünglichen Pfanne üblicherweise zerstörte Knochenmaterial ersetzen muss, da dieses nunmehr nicht mehr zur Halterung der Pfanne in der Hüfte zur Verfügung steht. So muss durch die Pfanne ein größerer Bereich abgedeckt werden als mit der Pfanne für die Erstversorgung, deren Außenumfangsfläche im Wesentlichen derjenigen einer Kugelzone entspricht, sodass die sphärische Außenfläche, die zwischen den oberseitigen flachen Abschlüssen der Kugelzone vorgesehen ist, streng rotationssymmetrisch ausgebildet ist.

Die sphärische Außenfläche einer Pfanne einer Revisionsprothese liegt zwar im Wesentlichen auch zwischen zwei abgeflachten und zumindest überwiegend parallel zueinander vorgesehenen Stirnseitenflächen, von denen die unteren Stirnseitenfläche eine Öffnung zu der inneren Aufnahme freilässt. Die sphärische Außenfläche ist aber nicht rotationssymmetrisch.

Dabei lässt sich bei üblichen Pfannen für die Endoprothetik Längsachse durch eine Bohrung legen, die als Montagehilfe für das Einbauen der Pfanne dient und mit einem Montagestab durchsetzt werden kann, um die Pfanne in dem Hüftknochen zu montieren. Rechtwinklig zu dieser Mittellängsachse erstrecken sich in der Regel die Stirnseitenflächen.

Die vorliegende Erfindung betrifft demnach eine Endoprothese, die bei ausgedehnten acetabularen Knochendefekten zur Anwendung kommt. Die zuvor beschriebene Pfanne der erfindungsgemäßen Revisionsendoprothese wird auch als "ovale" Pfanne bezeichnet. Diese Pfanne wird in dem Hüftknochen, d.h. dem Acetabulum über Press-fit fixiert.

Die vorliegende Erfindung will eine Revisionsendoprothese angeben, die das Risiko von Luxation des Hüftknochens verringert.

Zur Lösung dieses Problems wird mit der vorliegenden Erfindung eine Revisionsendoprothese mit den Merkmalen von Anspruch 1 vorgeschlagen. Die Revisionsendoprothese hat in an sich bekannter Weise eine ovale Pfanne, die eine innere Aufnahme zur verschwenkbaren Lagerung des Oberschenkelknochens ausformt. Die verschwenkbare Lagerung des Kugelkopfes, der mit dem Oberschenkelknochen verbunden ist, erfolgt aber nicht unmittelbar innerhalb dieser Aufnahme, sondern unter Zwischenlage eines Einsatzes mit einer sphärischen Außenfläche zur kugeligen Lagerung innerhalb der Aufnahme und einer sphärischen Innenfläche zur kugeligen Lagerung des Kugelkopfes.

Durch diese Ausgestaltung wird eine Aufteilung des Bewegungsumfangs auf zwei Artikulationsflächen erreicht. Die Artikulation findet bei der erfindungsgemäßen Revisionsprothese sowohl zwischen der acetabularseitig fest fixierten Pfanne und dem Einsatz einerseits wie auch zwischen dem Einsatz und dem Kugelkopf andererseits statt. Diese Aufteilung der sich ergebenden Relativbewegungen zwischen der Hüfte und dem Oberschenkelknochen entspricht in besonderer Weise dem Bedarf von Patienten, deren Endoprothese einmalig oder gar mehrmalig ausgetauscht wurde und die dementsprechend ausgedehnte Knochendefekte, insbesondere acetabulare Knochendefekte und Bandinstabilitäten haben. Für dieses Patientenklientel ist das Konzept einer zweifachen Beweglichkeit in Kombination mit einer ovalen Pfanne besonders geeignet.

Dabei kann die Pfanne für sich als einteiliger Körper ausgebildet sein, beispielsweise mittels Gießen, EBM bzw. SLM und eine sphärische Innenfläche zur kugeligen Lagerung des Einsatzes ausbilden. Üblicherweise und gemäß einer bevorzugten Weiterbildung der vorliegenden Erfindung wird indes ein Inlay vorgesehen, dass mit der Pfanne verbunden ist und eine sphärische Gegenfläche für die sphärische Außenfläche des Einsatzes ausbildet. Der Einsatz ist dementsprechend über das Inlay in der Pfanne kugelig gelagert. Das Inlay kann auf verschiedene Weise mit der Pfanne verbunden sein, beispielsweise verklebt, d.h. einzementiert oder mittels Schraub- und/oder Rastverbindung bzw. durch Klemmung also mittels Konusverbindung.

Gemäß einer bevorzugten Weiterbildung der vorliegenden Erfindung ist an der Pfanne zumindest ein von der Pfanne abragender Befestigungsflansch vorgesehen. Dieser Befestigungsflansch weist üblicherweise eine oder mehrere Bohrungen auf, welche von Schrauben durchsetzt werden kann, die acetabular verschraubt sind, um eine zuverlässige Fixierung der Pfanne zu ermöglichen.

Der Flansch kann dabei zusammen mit der Pfanne als einheitliches Bauteil ausgebildet sein. Alternativ kann der Flansch an einem umgeformten Blechelement vorgesehen sein, welches eine die Aufnahme überdeckende Kuppel aufweist und an einer Außenumfangsfläche mit einer das Knochenwachstum fördernden Oberflächengestaltung versehen ist. Eine solche Oberflächengestaltung kann mittels Verschweißen mit der Außenfläche des Blechelementes verbunden sein und beispielsweise durch ein Drahtgeflecht gebildet sein. Die Oberflächengestaltung kann auch in an sich bekannter Weise durch Plasmaspritzen aufgebracht bzw. mittels EBM bzw. SLM vorgesehen sein.

Auch auf dem einheitlichen Bauteil, welches den Flansch und die Pfanne ausformt, kann eine das Knochenwachstum fördernde Oberflächengestaltung der vorstehend genannten Art in der bereits zuvor beschriebenen Weise vorgesehen sein.

Weitere Einzelheiten und Vorteile der vorigen Erfindung ergeben sich aus der nachfolgenden Beschreibung eines Ausführungsbeispiels in Verbindung mit der Zeichnung. In dieser zeigen:
- Fig. 1: eine Schnittansicht eines Ausführungsbeispiels in Explosionsdarstellung;
- Fig. 2: die Pfanne des in Figur 1 gezeigten Ausführungsbeispiels in einer anderen Schnittansicht;
- Fig. 3: eine Seitenansicht und
- Fig. 4: eine Draufsicht auf eine ovale Pfanne eines anderen Ausführungsbeispiels.

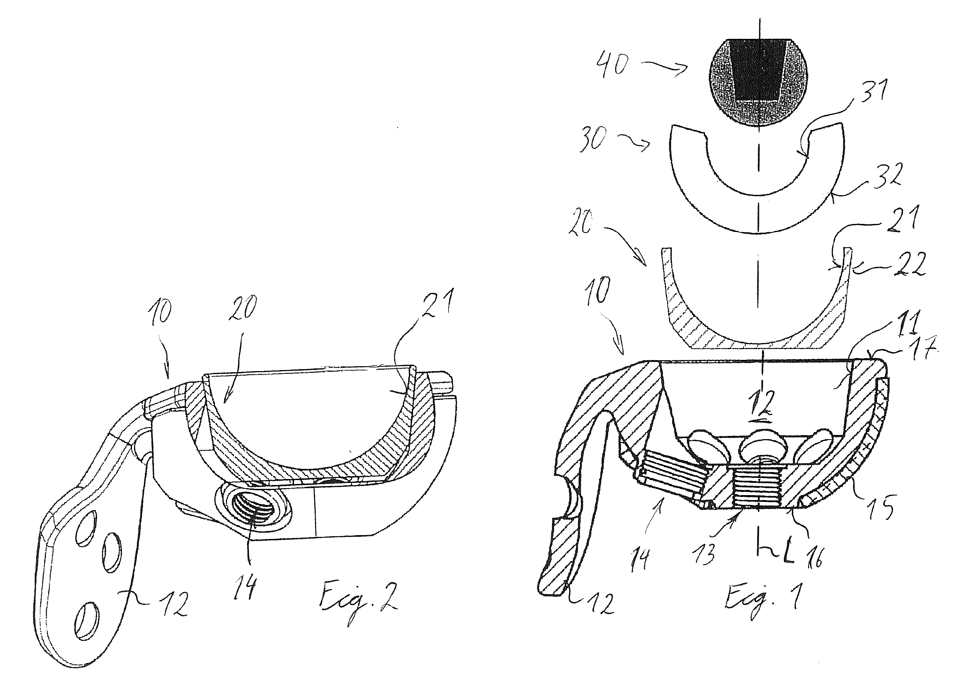

In der Längsschnittansicht nach Figur 1 kennzeichnet Bezugszeichen 10 eine Pfanne, Bezugszeichen 20 ein Metall-Inlay und Bezugszeichen 30 einen Einsatz. Diese Elemente bilden die Elemente der Revisionsendoprothese. Mit Bezugszeichen 40 ist ein Kugelkopf zeichnerisch ergänzt, der nach der endoprothetischen Versorgung des Patienten über einen Schaft mit dem Oberschenkel des Patienten verbunden ist und kugelig in einer In-nenumfangsfläche 31 des Einsatzes gelagert werden kann. Der Einsatz 30 besteht aus PE, insbesondere hochdichten PE (HDPE). Der Einsatz 30 hat eine sphärische Außenumfangsfläche 32, die kugelig an einer durch das Inlay 20 gebildeten sphärischen Gegenfläche 21 zur Anlage gelangt und dort kugelig gelagert ist. Das Inlay 20 ist aus Metall gebildet, beispielsweise Stahl und hat im Bereich der Öffnung zu der Gegenfläche 21 außenseitig einen Konusabschnitt 22, der mit einer konischen Gegenfläche 11, welche eine Aufnahme 12 der Pfanne 10 teilweise begrenzt, zusammenwirken kann, um das Inlay 20 mit der Pfanne 10 zu verbinden und verdrehfest daran zu sichern. Eine entsprechende Montage von Inlay 20 und Pfanne 10 ist in Figur 2 gezeigt. Wie im Übrigen die Figuren 1 und 2 verdeutlichen, weist die Pfanne 10 einen von ihrem sphärischen Grundkörper abragenden Flansch 12 auf, der mit mehreren Bohrungen versehen ist. Der Grundkörper der Pfanne 10 ist von einer mittleren Bohrung 13 mit Innengewinde durchsetzt, die eine Längsachse L definiert. Im Bereich der Kuppel und des Grundkörpers der Pfanne 10 sind weitere Bohrungen 14 vorgesehen, die jeweils mit einem Innengewinde versehen sind, um wahlweise eine kuppelseitig abschließende Abdeckkappe oder einen die Kuppel überragenden Befestigungszapfen zu montieren. Wie ferner die weitere Schraffur an der Außenfläche der Pfanne 10 verdeutlicht, ist diese mit einer das Knochenwachstum fördernden Oberflächengestaltung 15 versehen. Es handelt sich vorliegend um ein aufgeschweißtes Gitter, welches eine Vielzahl von Kavitäten zum Einwachsen von Knochenzellen bereitstellt. Diese Oberflächengestaltung 15 wird um die Längsachse L herum begrenzt von Material des Grundkörpers der Pfanne 10, welches eine erste kleine Stirnseitenfläche 16 ausbildet, die sich rechtswinklig zu der Mittellängsachse L erstreckt. Eine zweite Stirnseitenfläche 17 auf der gegenüberliegenden Seite begrenzt die Aufnahme 12.

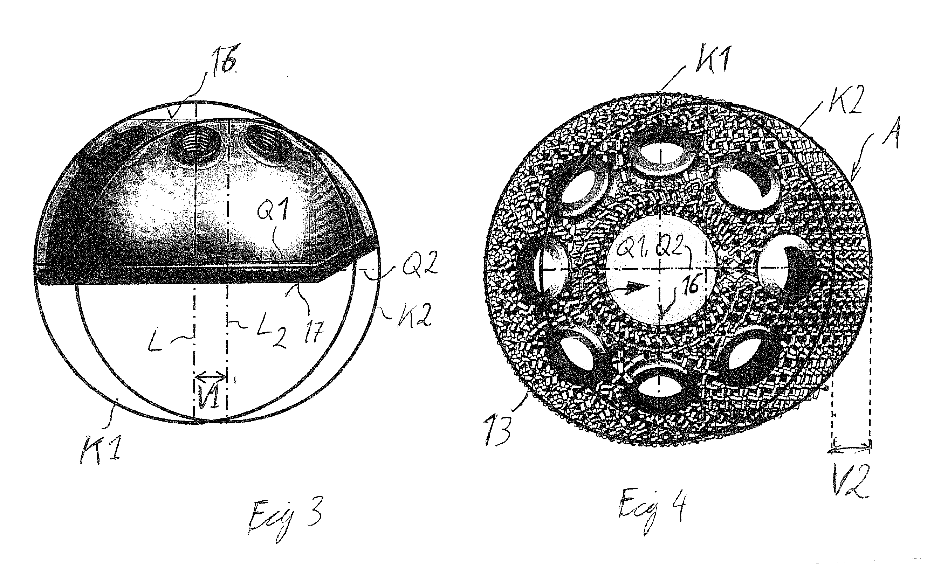

Die Figuren 3 und 4 verdeutlichen die ovale Form des Pfannengrundkörpers, d. h. der Pfanne ohne Flansch 12. Die Figur 3 zeigt dabei eine Seitenansicht in einer Ebene rechtwinklig zu der Ebene der Stirnseitenflächen 16 bzw. 17 und parallel zu der Längsachse L. Diese Längsachse L durchsetzt eine erste Querachse Q1. Der Schnittpunkt der beiden Linien L, Q1 ist der Mittelpunkt einer ersten Kugel K1 mit Durchmesser 56 mm. Parallel zu der Linie Q1 ist eine zweite Linie Q2 eingezeichnet, die in Richtung auf die größere Stirnseitenfläche 17 hin leicht gegenüber der Linie Q1 versetzt vorgesehen ist. Mit einem Versatz V1 von vorliegend 8 mm jedoch parallel zu der Längsachse L ist eine zweite Längsachse L2 eingezeichnet. Der Schnittpunkt L2, Q2 bildet den Mittelpunkt einer zweiten Kugel K2 mit einem Durchmesser von 52 mm. Teile der beiden Kugelflächen bilden die Außenfläche der Pfanne 10, die dementsprechend nicht rotationssymmetrisch ausgebildet ist, wie die Konturen K1, K2 in Fig. 3 und Fig. 4 belegen. Fig. 4 verdeutlicht dabei eine Draufsicht in einer Ebene rechtwinklig zu der Längsachse L und auf die kleinere Stirnseitenfläche 16 der Kugel 10. Durch diese Ausgestaltung ergibt sich ein ovales Design mit einem kranialen Aufbau A mit einem Versatz V2 von 6 mm gegenüber der Kugeloberfläche der Kugel K1. Bei dem in den Figuren 3 und 4 gezeigten Ausführungsbeispiel besteht der Grundkörper der Pfanne aus einem Cobald-Chrom-Molybdän-Gussmaterial, auf dem die Oberflächengestaltung 15 als TiNb-Beschichtung aufgebracht ist. Wie ersichtlich, sind in dieser Draufsicht die Achsen Q1, Q2 deckungsgleich, so dass der kraniale Aufbau A spiegelsymmetrisch zu den Querachsen Q1 bzw. Q2 ist.

### Bezugszeichenliste

- 10: Pfanne
- 11: Konusfläche
- 12: Flansch
- 13: mittlere Bohrung
- 14: seitliche Bohrungen
- 15: Knochenwachstumsfördernde Oberflächengestaltung
- 16: kleinere Stirnseitenfläche
- 17: größere Stirnseitenfläche
- 20: Inlay
- 21: Gegenfläche
- 22: Konusfläche
- 30: Einsatz
- 31: Innenumfangsfläche
- 32: Außenumfangsfläche
- 40: Kugelkopf
- A: kranialer Aufbau
- L: Längsachse
- L2: zweite Längsachse
- K1: erste Kugel
- K2: zweite Kugel
- Q1: erste Querachse
- Q2: zweite Querachse

## Patentansprüche

1. Revisionsendoprothese mit
einer Pfanne (10), die eine innere Aufnahme (13) zur verschwenkbaren Lagerung des Oberschenkelknochens ausformt und eine sphärische Außenfläche hat, die zumindest in einer Ebene nicht rotationssymmetrisch ist,
einem Einsatz (30) mit einer sphärischen Außenfläche (32) zur kugeligen Lagerung innerhalb der inneren Aufnahme (12) und einer sphärischen Innenfläche (31) zur kugeligen Lagerung eines Kugelkopfes (49), der mit dem Oberschenkelknochen verbunden ist.

2. Revisionsendoprothese nach Anspruch 1, **gekennzeichnet durch** ein Inlay (20), das mit der Pfanne (10) verbunden ist und eine sphärischen Gegenfläche (21) für die sphärische Außenfläche (32) des Einsatzes (30) ausbildet.

3. Revisionsendoprothese nach Anspruch 1 oder 2, **dadurch gekennzeichnet, dass** an der Pfanne (10) zumindest ein von der Pfanne (10) abragender Befestigungsflansch (12) vorgesehen ist.

4. Revisionsendoprothese nach Anspruch 3, **dadurch gekennzeichnet, dass** der Flansch (12) mit der Pfanne (10) ein einheitliches Bauteil ausbildet.

5. Revisionsendoprothese nach Anspruch 3, **dadurch gekennzeichnet, dass** der Flansch (12) an einem umgeformten Blechelement vorgesehen ist, welches eine die Aufnahme (12) überdeckende Kuppel aufweist und an seiner Außenumfangsfläche mit einer das Knochenwachstum fördernden Oberflächengestaltung (15) versehen ist.
